# EUROPEAN PATENT SPECIFICATION

(11) **EP 2 679 162 B1**
(45) Date of publication and mention of the grant of the patent: **31.08.2016**
(21) Application number: 12750291.2
(22) Date of filing: 20.01.2012
(51) Int. Cl.: A61B 8/00, G01N 29/24

(54) **ULTRASONIC PROBE USING A VERTICALLY ARRANGED MOTOR**
ULTRASCHALLSONDE MIT EINEM VERTIKAL ANGEORDNETEN MOTOR
SONDE ULTRASONIQUE UTILISANT UN MOTEUR ÉLECTRIQUE DISPOSÉ VERTICALEMENT

(30) Priority: 21.02.2011 KR 20110015289
(43) Date of publication of application: 01.01.2014
(73) Proprietor: Samsung Medison Co., Ltd., Gangwon-do 250-870 (KR)
(72) Inventor: SONG, In Seong, Gyeongju-si Gyeongbuk 780-905 (KR); OH, Won Gee, Gyeongju-si Gyeongbuk 780-905 (KR); SONG, In Jin, Gyeongju-si Gyeongbuk 780-905 (KR)
(74) Representative: Lorenz, Werner
(86) International application number: PCT/KR2012/000576
(87) International publication number: WO 2012/115354

(56) References cited:
- JP-A- 2006 110 972
- KR-A- 20080 063 694
- KR-A- 20110 010 283
- KR-B1- 100 393 354
- KR-B1- 100 455 606
- KR-B1- 100 455 606
- US-A- 5 377 682
- US-A- 5 781 007
- US-A1- 2002 062 080
- US-A1- 2006 074 316
- US-A1- 2008 161 694
- US-A1- 2010 076 316

## Description

### Technical Field

The present invention relates to an ultrasonic probe using a vertically arranged motor, and more particularly, to an ultrasonic probe using a vertically arranged motor, which has an ergonomic design by implementing a mechanism capable of a linear operation by providing a motor to stand upright in the ultrasonic probe.

### Background Art

An example of medical ultrasonic apparatuses includes an ultrasonic scanning apparatus mainly used to contrast an organ or a fetus in a human body. Unlike other medical apparatuses for contrasting the inside of a human body, such as X-ray imaging, computerized tomography (CT), and magnetic resonance imaging, the ultrasonic scanning apparatus may contrast a certain point inside the human body that is desired by a user as the user arbitrarily controls a radiation angle of ultrasonic waves. Also, not only the human body is not damaged by radiation, but also an image is obtained relatively quicker than other medical apparatuses for contrasting the inside of the human body.

In order to form an image by using the ultrasonic scanning apparatus, a unit and/or a device that mutually converts an ultrasonic signal and an electric signal is essential, and this unit and/or device is referred to as an ultrasonic probe or an ultrasonic transducer in the related art. The ultrasonic probe is typically formed of an ultrasonic module that includes: a piezoelectric layer that mutually converts an electric signal and a sound signal as a piezoelectric material is vibrated; a matching layer that reduces a difference in acoustic impedance between the piezoelectric layer and a human body so that ultrasonic waves generated in the piezoelectric layer are transmitted to a target portion of the human body as much as possible; a lens layer that focuses ultrasonic waves proceeding to the front of the piezoelectric layer, to a predetermined point; and a backing layer that blocks the ultrasonic waves from proceeding to the back of the piezoelectric layer to thereby prevent image distortion. Except for a single ultrasonic element designed for particular uses, a general medical ultrasonic probe includes a plurality of ultrasonic elements.

Such medical ultrasonic probes may be classified based on any one of various standards, such as the number of ultrasonic elements, an array method or a shape of an array axis of ultrasonic elements, or an application field. When the medical ultrasonic probes are classified according to the number of ultrasonic elements, the medical ultrasonic probes may be classified into single element type ultrasonic probes and plural element type ultrasonic probes. Here, the plural element type ultrasonic probes may be classified into one-dimensional (1 D) array ultrasonic probes, wherein ultrasonic elements are arranged on a single axis, and 2D array ultrasonic probes, wherein ultrasonic elements are arranged on a plurality of axes that cross each other, according to an array method of ultrasonic elements. Also, 1 D array ultrasonic probes may be classified into linear array ultrasonic probes and curvilinear array ultrasonic probes according to a shape of an array axis of ultrasonic elements.

1 D array ultrasonic probes that are mostly used are capable of realizing only a 2D image at a point in front of ultrasonic elements due to the linearity of ultrasonic waves. Accordingly, it is difficult to perform an accurate diagnosis by using general 1 D array ultrasonic probes, and it is not possible to contrast an overall figure of a fetus in 3D or contrast an appearance of a moving fetus in a moving image. Recently, ultrasonic probes capable of realizing a 3D image of inside a human body, in detail, a 3D dynamic image, have been requested, and a 3D image may be realized by using a general 1 D array ultrasonic probe or a 2D array ultrasonic probe.

However, it is difficult to manufacture a 2D array ultrasonic probe since the 2D array ultrasonic probe includes an extremely larger number of ultrasonic elements than a 1 D array ultrasonic probe. Also, an image obtained by the 2D array ultrasonic probe has a low quality due to a low signal-to-noise (S/N) ratio. Accordingly, a method of obtaining a 3D image by using a 1 D array ultrasonic probe has been continuously studied. KR-100 455 606 discloses an ultrasound probe of real-time 3D moving picture repeatedly swinging to the prescribed angle the array of ultrasonic elements arranged one-dimensionally by power generated from the motor.

In order to obtain a 3D image by using a 1 D array ultrasonic probe, a user moves the 1 D array ultrasonic probe manually or mechanically. When the 3D image is obtained as the user manually manipulates the 1 D array ultrasonic probe, the quality of the 3D image is remarkably low due to irregular constant intervals, and an error of the 3D image may be increased according to users. Thus, recently, a method of obtaining a 3D image by mechanically moving a 1 D array ultrasonic probe has been actively studied.

The method of obtaining a 3D image by mechanically moving a 1 D array ultrasonic probe may be performed by moving an array axis of ultrasonic elements in parallel or swinging an array axis of ultrasonic elements in a predetermined angle. When the array axis is moved in parallel, the array axis of the ultrasonic elements is moved to be parallel to a region of a human body to be contrasted by using a motor. Here, a contrast interval of the 3D image is maintained uniform and the occurrence of an error of the 3D image is reduced. However, in order to contrast a wide region of the human body, a size of an ultrasonic probe, including a power generating unit, such as the motor, has to be relatively large, and thus it is difficult to manufacture and use the ultrasonic probe.

When the array axis is swung, the array axis of the ultrasonic elements is swung in a predetermined angle above a region of a human body to be contrasted while moving an arc trajectory by using a power generating unit, such as a motor. Since a size of an ultrasonic probe may be relatively smaller than that when the array axis is moved in parallel, the usability of the ultrasonic probe may be excellent.

Ultrasonic probes used to obtain a 3D image by swinging an array axis of ultrasonic elements may be classified into mono-composition ultrasonic probes, wherein a module including ultrasonic elements and a power generating unit, such as a motor, are located in a single housing, and dual-composition ultrasonic probes, wherein a module and a power generating unit are not located in a single housing but are individually located. According to a dual-composition ultrasonic probe, a module and a power generating unit that are not located within a same housing are coupled to each other by a separate component. Here, a 2D image may be obtained by using a general 1 D array ultrasonic probe, but since the power generating unit independently exists from the module, a size of an ultrasonic probe is relatively larger than a mono-composition ultrasonic probe, and thus the usability of the dual-composition ultrasonic probe is low. In addition, since a portion that actually swings with the module contacts a human body and presses a surface of the human body, a quality of the 2D image may be lower than the mono-composition ultrasonic probe.

According to a mono-composition ultrasonic probe, a module and a power generating unit are located in a same housing, and thus a size of an ultrasonic probe may be relatively smaller than a dual-composition ultrasonic probe. Also, since a change of a surface of a human body that occurs when the ultrasonic probe is used is remarkably reduced, the mono-composition ultrasonic probe may be very conveniently used.

However, the producibility and durability of a general mono-composition ultrasonic probe are low since a mechanical driving relation for swinging an array axis of ultrasonic elements in a predetermined angle is complex. When the array axis is swung in a plurality of swing operations within a predetermined swing angle in order to obtain a high-quality image, the swing operations are controlled only by a motor driver. Thus, in order to increase the number of swing operations, a motor or the motor driver is replaced or a performance of the motor driver is increased through software. Accordingly, manufacturing costs are increased and utilization is decreased.

### Disclosure of the Invention

### Technical Problem

The present invention is defined by the appended claims. It provides an ultrasonic probe using a vertically arranged motor, wherein the motor stands upright in the ultrasonic probe and a side pulley is located in a tangential direction of a probe-shaft pulley, thereby enabling a rotating force generated by the vertically arranged motor to be smoothly transferred to an array axis of ultrasonic elements and significantly simplifying a structure of the ultrasonic probe to remarkably reduce manufacturing costs.

The present invention also provides an ultrasonic probe using a vertically arranged motor, which has an ergonomic design to significantly improve user satisfaction by implementing a mechanism which enables a linear operation through use of the vertically arranged motor.

### Technical Solution

According to an aspect of the present invention, there is provided
an ultrasonic probe using a vertically arranged motor, wherein a 3 dimensional (3D) image is obtained by swinging in a predetermined angle, an array axis of a plurality of ultrasonic transmission and reception elements that are one-dimensionally arranged, the ultrasonic probe including: a rotation motor that stands upright and generates a rotating force; two side pulleys that convert a rotation direction of the rotating force by receiving the rotating force from the rotation motor and transfer the rotating force having the converted rotation direction to a probe-shaft pulley; the probe-shaft pulley that rotates the array axis of the plurality of ultrasonic transmission and reception elements via the rotating force received from the two side pulleys; and a wire that transfers the rotating force by being wound on a rotation shaft of the rotation motor, the side pulley, and the probe-shaft pulley, wherein the two side pulleys are disposed to be parallel in a tangential direction of the probe-shaft pulley.

The wire may be wound on the rotation shaft of the rotation motor at least once.

The wire may be wound on the rotation shaft of the rotation motor in a spiral form.

The two side pulleys may be respectively disposed on two sides of the rotation motor

The two side pulleys may be rotated by the wire wound on the probe-shaft pulley while being located on a plane vertical to a plane where the probe-shaft pulley is placed.

The two side pulleys may be rotated based on an axis at a predetermined angle from the rotation shaft of the rotation motor.

Each of the two side pulleys or the probe-shaft pulley may include a groove for preventing the wire from deviating, at an edge.

### Effects of the Invention

According to an ultrasonic probe using a vertically arranged motor, a motor stands upright in the ultrasonic probe and a side pulley is located in a tangential direction of a probe-shaft pulley, thereby enabling a rotating force generated by the vertically arranged motor to be smoothly transferred to an array axis of ultrasonic elements and significantly simplifying a structure of the ultrasonic probe to remarkably reduce manufacturing costs.

In addition, according to the ultrasonic probe using the vertically arranged motor, a mechanism which enables a linear operation is implemented while using the vertically arranged motor. Thus, the ultrasonic probe may have an ergonomic design to significantly improve user satisfaction.

### Brief Description of the Drawings

FIG. 1 is a cross-sectional view of a general ultrasonic probe;
FIG. 2 is a perspective view of an ultrasonic probe using a vertically arranged motor, according to an embodiment of the present invention;
FIG. 3 is a diagram of an internal structure of an ultrasonic probe using a vertically arranged motor, according to an embodiment of the present invention;
FIG. 4 is a diagram of an internal structure of a general ultrasonic probe using a vertically arranged motor; and
FIG. 5 is graphs for comparing a swing angle of a general ultrasonic probe and a swing angle of an ultrasonic probe using a vertically arranged motor, according to an embodiment of the present invention.

**<Explanation of Reference numerals designating the Major Elements of the Drawings>**

| | |
|---|---|
| 100: Ultrasonic Probe using Vertically Arranged Motor | |
| 110: Rotation Motor | 120: Side Pulley |
| 130: Probe-Shaft Pulley | 140: Wire |
| 200: General Ultrasonic Probe using Vertically Arranged Motor | |
| 210: Rotation Motor | 220: Side Pulley |
| 230: Probe-Shaft Pulley | 240: Wire |

### Best Mode for carrying out the Invention

Hereinafter, the present invention will be described more fully with reference to the accompanying drawings, in which exemplary embodiments of the invention are shown. While describing the present invention, detailed descriptions about related well-known functions or configurations that may diminish the clarity of the points of the present invention are omitted. In the drawings, like reference numerals denote like elements.

It will be understood that when a portion is referred to as being "connected to" another region, it can be "directly connected to" the other region, or "indirectly connected" with an intervening elements therebetween.

Also, when an element is "included", another element may be further included unless stated otherwise.

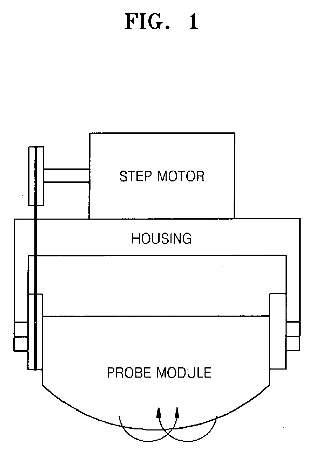

FIG. 1 is a cross-sectional view of a general ultrasonic probe. As shown in FIG. 1, the general ultrasonic probe includes a step motor in a lateral direction to swing an array axis of ultrasonic transmission and reception elements, wherein a motor-shaft pulley and a probe-shaft pulley are connected to the step motor via a wire. Here, since the step motor is disposed in the lateral direction, an overall shape of the general ultrasonic probe is limited, and thus the general ultrasonic probe is difficult to be used.

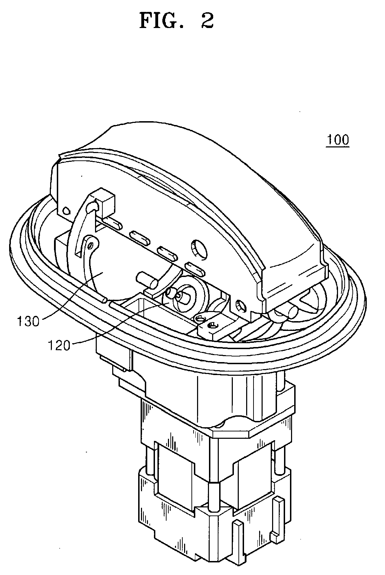

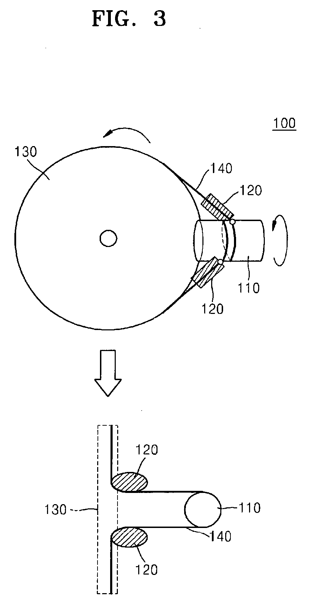

FIG. 2 is a perspective view of an ultrasonic probe 100 using a vertically arranged motor, according to an embodiment of the present invention, and FIG. 3 is a diagram of an internal structure of the ultrasonic probe 100, according to an embodiment of the present invention. An upper drawing in FIG. 3 is a front view of the internal structure and a lower drawing in FIG. 3 is a plan view of the internal structure. As shown in FIGS. 2 and 3, the ultrasonic probe 100 that obtains a 3 dimensional (3D) image by swinging in a predetermined angle, an array axis of a plurality of ultrasonic transmission and reception elements that are one-dimensionally arranged may include a rotation motor 110, a side pulley 120, a probe-shaft pulley 130, and a wire 140.

The rotation motor 110 stands upright and generates a rotating force. Since the rotation motor 110 stands upright, the ultrasonic probe 100 may have an ergonomic design. Of course, the array axis of the ultrasonic transmission and reception elements may move linearly as will be described below even when the rotation motor 110 stands upright.

Here, providing the rotation motor 110 to stand upright means that the rotation motor 110 is provided in a direction of transmitting and receiving ultrasonic waves, and a rotation direction of the rotating force generated by the rotation motor 110 is converted by the side pulley 120 and then is transferred to the probe-shaft pulley 130 that rotates based on an axis vertical to a rotation shaft of the rotation motor 110.

Two side pulleys 120 may be used, and may convert the rotation direction of the rotating force upon receiving the rotating force from the rotation motor 110 and transmit the rotating force having the converted rotation direction to the probe-shaft pulley 130. The two side pulleys 120 may be respectively disposed on both sides of the rotation motor 110, and smoothly transfer the rotating force of the rotation motor 110 through the wire 140. Also, the two side pulleys 120 may be disposed in parallel to a tangential direction of the probe-shaft pulley 130 in order to suitably change the rotation direction of the rotating force.

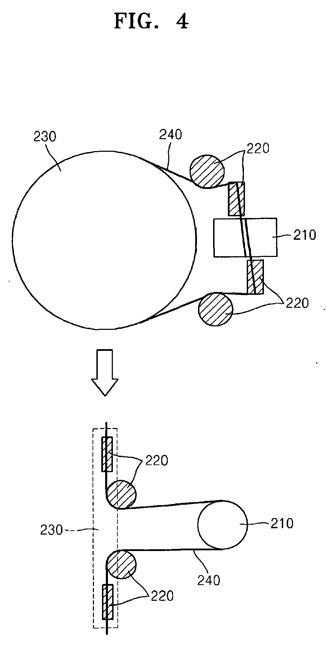

In other words, the side pulley 120 may be rotated by the wire 140 wound on the probe-shaft pulley 130 while being placed on a plane vertical to a plane where the probe-shaft pulley 130 is placed, and may rotate based on an axis at a predetermined angle from the rotation shaft of the rotation motor 110. As such, by disposing the side pulley 120 to be misaligned from the probe-shaft pulley 130 and the rotation motor 110, the rotating force of the rotation motor 110 may be transferred to the probe-shaft pulley 130 after converting the rotation direction of the rotating force to be vertical. Effects obtained via the misaligned arrangement of the side pulley 120 will now be described in detail with reference to FIG. 4.

FIG. 4 is a diagram of an internal structure of a general ultrasonic probe 200 using a vertically arranged motor. An upper drawing of FIG. 4 is a front view of the internal structure and a lower drawing of FIG. 4 is a plan view of the internal structure. As shown in FIG. 4, in the general ultrasonic probe 200, a total of four side pulleys 220 are used to smoothly transfer a rotating force from a rotation motor 210 to a probe-shaft pulley 230. However, such an internal structure is very complex, and thus manufacturing costs are increased.

However, according to the ultrasonic probe 100, since the rotating force is transferred to the probe-shaft pulley 130 by using the two side pulleys 120 that are misaligned from the probe-shaft pulley 130 but are disposed in parallel on a tangential line of the probe-shaft pulley 130 as described above, the number of side pulleys 120 may be reduced, and the rotating force may be effectively prevented from being unnecessarily reduced due to friction with the wire 140 while transferring the rotating force.

The probe-shaft pulley 130 rotates the array axis of the ultrasonic transmission and reception elements via the rotating force received from the side pulley 120.

Since the ultrasonic probe 100 is an ultrasonic probe that obtains a 3D image by swinging the array axis of the ultrasonic transmission and reception elements that are one-dimensionally arranged, the rotating force generated by the rotation motor 110 may be transferred to the probe-shaft pulley 130 for a swing motion. Here, as described above, by particularly disposing the side pulleys 120 required to transmit the rotating force, a structure of the ultrasonic probe 100 may be simplified, thereby reducing the manufacturing costs while increasing the transmission efficiency of the rotating force.

Here, a groove (not shown) for preventing the wire 140 from deviating may be formed at an edge of the probe-shaft pulley 130. In other words, in the ultrasonic probe 100, the wire 140 moves while being inserted into the groove of the probe-shaft pulley 130 so that the rotating force is transferred to the probe-shaft pulley 130 via a friction generated between the wire 140 and the probe-shaft pulley 130, while preventing the wire 140 from being separated from the probe-shaft pulley 130 by sliding during an operation. Here, the groove for preventing the wire 140 from deviating may not only formed at the edge of the probe-shaft pulley 130, but also at the edge of the side pulley 120.

The wire 140 transfers the rotating force by being wound on the rotation shaft of the rotation motor 110, the side pulley 120, and the probe-shaft pulley 130. Since the rotating force is transferred via friction, the wire 140 may be formed of a material capable of generating enough frictional force not to slide from surfaces of the probe-shaft pulley 130 and the side pulley 120.

The wire 140 may be wound on the rotation shaft of the rotation motor 110 at least once so that the rotating force of the rotation motor 110 is transferred to the probe-shaft pulley 130 without any loss. Here, the wire 140 may be wound on the rotation shaft of the rotation motor 110 in a spiral form so that the wire 140 that is wound a plurality of times does not interfere with itself, and thus heights of the side pulleys 120 on the both sides of the rotation motor 110 may be different. In other words, the wire 140 wound on the side pulley 120 on the left of the rotation shaft may be wound on the rotation shaft in a spiral form and come out at the right of the rotation shaft, and thus the side pulley 120 on the right of the rotation shaft may be provided lower than the side pulley 120 on the left of the rotation shaft. However, locations of the side pulleys 120 are not limited thereto, and a loss of the rotating force may be sufficiently prevented as long as the heights of the side pulleys 120 are different.

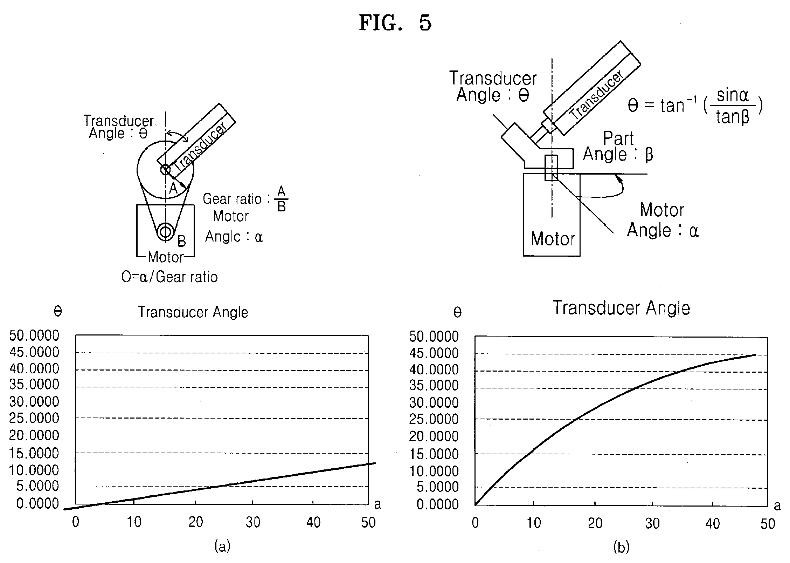

FIG. 5 is graphs for comparing a swing angle of a general ultrasonic probe and a swing angle of the ultrasonic probe 100, according to an embodiment of the present invention. As shown in FIG. 5(a), in the general ultrasonic probe wherein a rotation motor is disposed in a lateral direction, a swing angle θ of an array axis of ultrasonic transmission and reception elements is increased as a rotation angle α of the rotation motor is increased, but since an increasing rate is low, a wide swing is not possible. On the other hand, as shown in FIG. 5(b), in the ultrasonic probe 100, by providing the rotation motor 110 to stand upright, a swing angle θ of an array axis of ultrasonic transmission and reception elements is remarkably increased when a rotation angle α of the rotation motor 110 is increased. This means that the array axis of the ultrasonic transmission and reception elements may swing in a wider range than the general ultrasonic probe, and thus a user may further conveniently scan a body of a patient compared to the general ultrasonic probe, thereby remarkably increasing user convenience.

While the present invention has been particularly shown and described with reference to exemplary embodiments thereof, it will be understood by those of ordinary skill in the art that various changes in form and details may be made therein without departing from the scope of the present invention as defined by the following claims.

## Claims

1. An ultrasonic probe (100) using a vertically arranged motor, wherein a 3 dimensional (3D) image is obtained by swinging in a predetermined angle, an array axis of a plurality of ultrasonic transmission and reception elements that are one-dimensionally arranged, the ultrasonic probe comprising:
a rotation motor (110) that generates a rotating force;
two side pulleys (120) that convert a rotation direction of the rotating force by receiving the rotating force from the rotation motor (110) and transfer the rotating force having the converted rotation direction to a probe-shaft pulley (130);
the probe-shaft pulley (130) that rotates the array axis of the plurality of ultrasonic transmission and reception elements via the rotating force received from the two side pulleys (120); and
a wire (140) that transfers the rotating force by being wound on a rotation shaft of the rotation motor (110), the two side pulleys (120), and the probe-shaft pulley (130),
wherein the two side pulleys (120) are respectively disposed on two sides of the rotation motor (110), are rotated by the wire (140) wound on the probe-shaft pulley (130), and **characterised in that** the two side pulleys are disposed to be parallel to a tangential direction of the probe-shaft pulley (130) and are rotated based on an axis at a predetermined angle from the rotation shaft of the rotation motor, and an axis of the probe-shaft pulley (130) is vertical to a plane where axes of the two side pulleys (120) are placed.

2. The ultrasonic probe (100) of claim 1, wherein the wire (140) is wound on the rotation shaft of the rotation motor (110) at least once.

3. The ultrasonic probe (100) of claim 2, wherein the wire (140) is wound on the rotation shaft of the rotation motor (110) in a spiral form.

4. The ultrasonic probe (100) of claim 1, wherein each of the two side pulleys (120) or the probe-shaft pulley (130) comprises a groove for preventing the wire (140) from deviating, at an edge.

## Patentansprüche

1. Ultraschallsonde (100), welche einen senkrecht angeordneten Motor verwendet, wobei ein 3-Dimensionales (3D)-Bild durch Schwingen einer Array-Achse einer Vielzahl von Ultraschall-Übertragungs- und Aufnahmeelementen erhalten wird, die Ein-Dimensional angeordnet sind, wobei die Ultraschallsonde folgendes aufweist:
Einen Drehmotor (110), der eine Rotationskraft erzeugt;
Zwei seitliche Rollen (120), welche eine Rotationsrichtung der Rotationskraft durch Aufnehmen der Rotationskraft von dem Drehmotor (110) konvertieren und die Rotationskraft, welche die konvertierte Rotationsrichtung aufweist, zu einer Sondenwellenrolle (130) übertragen;
Die Sondenwellenrolle (130), welche die Array-Achse der Vielzahl von Ultraschall-Übertragungs- und Aufnahmeelementen über die von den zwei seitlichen Rollen (120) aufgenommene Rotationskraft rotiert; und
ein Kabel (140), welches die Rotationskraft dadurch überträgt, dass es auf einer Rotationswelle des Drehmotors (110), den zwei seitlichen Rollen (120) und der Sondenwellenrolle (130) aufgewickelt ist,
wobei die zwei seitlichen Rollen (120) jeweils auf zwei Seiten des Drehmotors (110) angeordnet sind und durch das Kabel (140), das auf der Sondenwellenrolle (130) aufgewickelt ist, rotiert werden,
**dadurch gekennzeichnet, dass**
die zwei seitlichen Rollen so angeordnet sind, dass sie parallel zu einer tangentialen Richtung der Sondenwellenrolle (130) sind und basierend auf einer Achse mit einem vorbestimmten Winkel von der Rotationswelle des Drehmotors rotiert werden, und eine Achse der Sondenwellenrolle (130) vertikal zu einer Ebene ist, in der Achsen der zwei seitlichen Rollen (120) angeordnet sind.

2. Ultraschallsonde (100) nach Anspruch 1, wobei das Kabel (140) wenigstens einmal auf der Rotationswelle des Drehmotors (110) gewickelt ist.

3. Ultraschallsonde (100) nach Anspruch 2, wobei das Kabel (140) in einer Spiralform auf der Rotationswelle des Drehmotors (110) gewickelt ist.

4. Ultraschallsonde (100) nach Anspruch 1, wobei jede der zwei seitlichen Rollen (120) oder die Sondenwellenrolle (130) an einem Rand eine Nut aufweist, um zu verhindern, dass das Kabel (140) abweicht.

## Revendications

1. Sonde ultrasonique (100) utilisant un moteur disposé verticalement, dans laquelle on obtient une image à trois dimensions (3D) en pivotant dans un angle prédéterminé, sur une zone axiale d'une pluralité d'éléments ultrasoniques de transmission et de réception qui sont disposés de façon monodimensionnelle, la sonde ultrasonique comportant :
un moteur rotatif (110) qui engendre une force de rotation ;
deux poulies latérales (120) qui convertissent une direction de rotation de la force de rotation en recevant la force de rotation du moteur rotatif (110) et qui transfère la force de rotation ayant la direction de rotation convertie à une poulie d'arbre (130) d'une sonde ;
la poulie d'arbre (130) de la sonde qui tourne sur la zone axiale de la pluralité d'éléments ultrasoniques de transmission et de réception via la force de rotation reçue des deux poulies latérales (120) ; et
un câble (140) qui transfère la force de rotation en étant enroulé sur un arbre rotatif du moteur rotatif (110), les deux poulies latérales (120), et la poulie d'arbre (130) de la sonde,
dans laquelle les deux poulies latérales (120), sont respectivement disposées sur deux côtés du moteur rotatif (110), pour être parallèles à une direction tangentielle de la poulie d'arbre (130) de la sonde, sont entraînées en rotation par le câble (140) enroulé sur la poulie d'arbre (130) de la sonde, et **caractérisée en ce que** les deux poulies latérales sont disposées et sont entraînées en rotation selon un axe ayant un angle prédéterminé par rapport à l'angle de l'arbre (130) de rotation du moteur rotatif, et un axe de la poulie d'arbre (130) de la sonde est perpendiculaire à un plan sur lequel les axes de deux poulies latérales (120) sont placées.

2. Sonde ultrasonique (100) selon la revendication 1, dans laquelle le câble (140) est enroulé au moins une fois sur l'arbre de rotation du moteur rotatif (110).

3. Sonde ultrasonique (100) selon la revendication 2, dans laquelle le câble (140) est enroulé sur l'arbre de rotation du moteur rotatif (110) sous la forme d'une spirale.

4. Sonde ultrasonique (100) selon la revendication 1, dans laquelle chacune des deux poulies latérales (120) ou la poulie d'arbre (130) de la sonde comporte une gorge pour empêcher la câble (140) de dévier sur un bord.
